# EUROPÄISCHE PATENTSCHRIFT

(11) **EP 2 407 105 B1**
(45) Veröffentlichungstag und Bekanntmachung des Hinweises auf die Patenterteilung: **07.09.2022**
(21) Anmeldenummer: 11172370.6
(22) Anmeldetag: 01.07.2011
(51) Int. Cl.: A61B 5/145

(54) **Selbstreinigende Sensoroberflächen für implantierbare Sensorsysteme**
Self-cleaning sensor surfaces for implantable sensor systems
Surface de détection auto-nettoyantes pour systèmes de détection implantables

(30) Priorität: 14.07.2010 US 364029 P
(43) Veröffentlichungstag der Anmeldung: 18.01.2012
(73) Patentinhaber: Dyconex AG, 8303 Bassersdorf (CH)
(72) Erfinder: Hauer, Marc, 8050 Zürich (CH); Dörr, Thomas, 12437 Berlin (DE)
(74) Vertreter: Biotronik Corporate Services SE

(56) Entgegenhaltungen:
- US-A1- 2003 114 735
- US-A1- 2005 096 587
- US-A1- 2006 257 996
- US-B2- 6 702 857

## Beschreibung

Implantierbare Sensorsysteme sind bekannt und werden beispielsweise in der Medizin eingesetzt, um bestimmte medizinisch relevante Parameter im menschlichen Körper zu erfassen. Meist werden durch solche Sensorsysteme Parameter in Körperflüssigkeiten über einen bestimmten Zeitraum hinweg erfasst. Dazu werden die Sensorsysteme derart implantiert, dass das Sensorsystem mit der Körperflüssigkeit, die den zu messenden Parameter enthält, in funktionalem Kontakt steht, so dass der Sensor den zu messenden Parameter erfassen kann.

Ein Problem bei implantierten Sensorsystemen ist, dass diese innerhalb einer gewissen Zeit nach der Implantation im Körper als Fremdkörper erkannt und verkapselt werden und dadurch von den Körperflüssigkeiten entkoppelt werden. Diese Entkopplung führt dazu, dass der funktionale Kontakt zwischen Körperflüssigkeit und Sensorsystem beeinträchtigt und gegebenenfalls sogar unterbrochen wird. Ein Problem bei implantierten Sensorsystemen ist, dass diese innerhalb einer gewissen Zeit nach der Implantation im Körper als Fremdkörper erkannt und verkapselt werden und dadurch von den Körperflüssigkeiten entkoppelt werden. Diese Entkopplung führt dazu, dass der funktionale Kontakt zwischen Körperflüssigkeit und Sensorsystem beeinträchtigt und gegebenenfalls sogar unterbrochen wird.

Der erste Schritt der Verkapselung besteht in der Anlagerung von körpereigenem Material an der Oberfläche des implantierten Sensorsystems. Später erfolgen aufbauend auf die erfolgte Anlagerung von körpereigenem Material die Umwachsung und der Einschluss des Sensorsystems durch Bindegewebszellen. (siehe James M. Anderson, Analiz Rodriguez and David T. Chang: Foreign body reaction to biomaterials; Seminars in Immunology Volume 20, Issue 2, April 2008, Pages 86-100 Innate and Adaptive Immune Responses in Tissue Engineering).

Es sind bereits Ansätze bekannt, die auf eine Verlängerung der Lebenszeit von implantierten Sensorsystemen gerichtet sind. So gibt es beispielsweise Sensorsysteme, die eine Vielzahl von eigenständigen Sensoren in einem Sensorarray angeordnet aufweisen, wobei die einzelnen Sensoren während des Betriebs des Sensorsystems koordiniert über die Zeit freigeschaltet werden. Bei dieser Herangehensweise wird also die Verkapselung nicht direkt verhindert, sondern nach erfolgter Verkapselung eines ersten Sensors des Sensorsystems wird einfach ein anderer, noch nicht verkapselter Sensor in Betrieb genommen. Ein Problem dieses Ansatzes liegt darin, dass diese Sensorsysteme naturgemäß relativ groß und komplex ausgebildet sein müssen, da sie mehrere Sensoren für einen sequentiellen Betrieb beherbergen müssen. Dies führt neben den räumlichen Anforderungen auch zu nicht unerheblichen Mehrkosten.

Alternativ sind auch Beschichtungen vorgeschlagen worden, die gezielt bestimmte biochemische Prozesse der Anlagerungsreaktionen und -prozesse unterbinden oder stören sollen. Diese Beschichtungen interagieren allerdings meist nur mit einem bestimmten oder mit wenigen ausgewählten Schritten des Verkapselungsprozesses. Da eine Verkapselung durch verschiedene ggf. alternative Prozesse oder Prozessschritte erfolgen kann, ist die erreichte Beeinflussung meist in ihrem Ausmaß beschränkt. Nach einer anfänglichen Verzögerung kommt es in der Regel doch zu einer die Lebenszeit des Sensorsystems beschränkenden Verkapselung.

US 2006/257996 A1 offenbart einen implantierbaren Analyt-Sensor, welcher eine elektrische nicht leitfähige, biokompatible Matrix aufweist, wobei die Matrix eine Vielzahl von Durchgängen umfasst. Der Analyt-Sensor umfasst weiterhin eine Arbeitselektrode, dessen elektroaktive Oberfläche innerhalb der Matrix ist. Die Matrix ist hierbei insbesondere als zellstörende Barriere ausgebildet.

US 2003/0114735 A1 offenbart einen Sensor zur Implantation in ein Blutgefäß und zu Beobachtung eines Analyten im Blut. Zur Reduzierung der Verkapslung wird ein stromlinienförmiges Sensorgehäuse vorgeschlagen, an dessen Spitze die Sensoroberfläche angeordnet ist. Des Weiteren ist insbesondere eine für den Analyten durchlässige Membran vorgesehen, welche das Sensorgehäuse bedeckt.

US 2005/096587 A1 offenbart einen implantierbaren Sensor, wobei der Sensor in einem Reservoir vorliegen kann, welches durch eine Kappe geschützt ist. Die Kappe kann aus einem biokompatiblen Material bestehen und dazu ausgebildet sein, sich kontrolliert aufzulösen, um so den Sensor der Zielumgebung zu exponieren.

US 6,702,857 offenbart eine Biointerfacemembran zur Verwendung mit einem implantierbaren Medizingerät, wobei die Biointerfacemembran dazu ausgebildet ist, die Bildung einer Zellschichtbarriere zu stören.

Aufgabe der vorliegenden Erfindung ist es einen oder mehrere Nachteile des Standes der Technik zu vermindern oder zu vermeiden. Insbesondere ist es eine Aufgabe der vorliegenden Erfindung, ein verbessertes implantierbares Sensorsystems zur Verfügung zu stellen, durch welches eine vorzeitige Verkapselung des Sensorsystems verzögert oder verhindert werden kann.

Die Aufgabe wird gelöst durch Bereitstellung eines implantierbaren Sensorsystems umfassend einen Sensor, der in einem Gehäuse angeordnet ist, wobei das Gehäuse einen Messbereich aufweist, der durchlässig ist für die vom Sensor zu erfassenden Parameter, dadurch gekennzeichnet, dass der Messbereich eine erodierbare Schutzbeschichtung aufweist, die durchlässig ist für die vom Sensor zu erfassenden Parameter. Bevorzugt ist die erodierbare Schutzbeschichtung mindestens auf der dem Sensor abgewandten Seite des Messbereichs angebracht.

Der Erfindung liegt die überraschende Erkenntnis zugrunde, dass sich die für den Beginn einer Verkapselungsreaktion notwendige Ablagerung von körpereigenen Materialien dadurch verringern oder verzögern lässt, dass das Sensorsystem eine erodierbare Schutzbeschichtung aufweist. Von dieser erodierbaren Schutzbeschichtung lösen sich kontrolliert Materialschichten in definierten Zeiträumen von der Sensoroberfläche. Dadurch wird erreicht, dass bereits erfolgte Ablagerungen auf der Oberfläche des Sensorsystems gelöst und entfernt werden. Die angewandte Vorgehensweise ist also unabhängig von den unterschiedlichen Schritten und Prozessen einer Verkapselungsreaktion und führt zu einer effektiven Verzögerung oder Verhinderung einer Verkapselung.

Das erfindungsgemäße Sensorsystem weist einen Sensor auf. Unter einem Sensor wird dabei ein technisches Bauteil verstanden, das bestimmte physikalische, chemische oder biochemische Eigenschaften oder Parameter und/oder eine stoffliche Beschaffenheit der Umgebung qualitativ oder als Messgröße quantitativ erfassen kann. Die Messgrößen werden meist mittels physikalischer oder chemischer Effekte erfasst und in weiterverarbeitbare Größen umgeformt, z.B. in elektrische Signale, die dann ggf. über weitere Bestandteile des Sensors an einem in der Umgebung befindlichen Empfänger übermittelt werden können.

Der Sensor kann beispielsweise eine Spule umfassen zur telemetrischen Kommunikation. Mittels dieser telemetrischen Kommunikation ist es beispielsweise möglich das Sensorsystem nach erfolgter Implantation von außerhalb des Körpers des Empfängers zu aktivieren, zu deaktivieren, zu regeln und/oder zu steuern sowie Messergebnisse zu übertragen.

Zum Schutz vor Umwelteinflüssen ist der Sensor in einem Gehäuse angeordnet, welches den Sensor im Wesentlichen umschließt. Das Gehäuse kann dabei flexibel gestaltet oder als starres Gebilde ausgebildet sein. Bevorzugt umfasst oder besteht das Gehäuse aus einem nicht-erodierbaren Werkstoff. Unter einem nicht-erodierbaren Werkstoff wird ein Werkstoff verstanden, der im Wesentlichen beständig ist gegenüber den Bedingungen, des Millieus und/oder der Umgebung nach erfolgter Implantation des Sensorsystems für einen Zeitraum, der mindestens der geplanten Standzeit, Messzeitraum oder des Erfassungszeitraums entspricht. Der nicht-erodierbarer Werkstoff ist bevorzugt biokompatibel und im Wesentlichen nicht biokorrodierbar. Bei dem nicht-erodierbaren Werkstoff handelt es sich bevorzugt um einen polymeren, einen metallischen, einen keramischen Werkstoff und/oder um eine Mischung enthaltend oder bestehend aus mehreren gleichen oder verschiedenen solcher Werkstoffe wie beispielsweise medizinischer Edelstahl (MP35N), Titan, Platin/Iridium-Legierung, Gold, Polyurethan, Silikon, Epoxidharz, Liquid Crystal Polymer (LCP) und dergleichen.

Um einen Kontakt zwischen dem im Gehäuse befindlichen Sensor und den zu erfassenden Parametern zu erlauben, der notwendig ist, damit der Sensor seinen Zweck erfüllen kann, ist im Gehäuse ein Messbereich vorgesehen. Der Messbereich zeichnet sich dadurch aus, dass er durchlässig ist für die vom Sensor zu erfassenden Parameter. Dazu kann der Messbereich als Aussparung oder Fenster im Gehäuse ausgestaltet sein, durch welches ein Kontakt zwischen Sensor und zu erfassenden Parameter möglich ist. Der Messbereich kann auch als Teil der Oberfläche des Gehäuses ausgestaltet sein, der einen Werkstoff aufweist oder daraus besteht, der durchlässig ist für die vom Sensor zu erfassenden Parameter, während er für andere Umweltparameter nicht permeabel ist. Eine entsprechende Durchlässigkeit für die zu bestimmenden Parameter kann auch dadurch erreicht werden, dass das Gehäuse im Messbereich eine perforierte Oberfläche aufweist, z.B. eine perforierte Membran, wobei die Öffnungen der Perforation bevorzugt so gewählt sind, dass die zu erfassenden Parameter diese passieren können. Bevorzugte Werkstoffe für solche Membrane im Messbereich sind beispielsweise geätzte Gold- oder Edelstahlfolien, perforierte LCP-Folien, Membranen aus Poly-L-Lactid oder einem weiteren Vertreter der Polyester wie PDLLA, PLGA, P3HB,P4HB oder Gemischen oder Copolymeren daraus, Parylen (Parylen C oder andere Derivate) bevorzugt als Parylen mit Pinholes, Cellulosefilme wie beispielsweise Nitrocellulose, Methylcellulose, Carboxymethylcellulose oder Polyvinylalkohol, wobei durch Molmasse und Deacetyliierungsgrad die Filmbildung optimiert werden kann.

Das Gehäuse des Sensorsystems kann ebenfalls durchlässig sein für die vom Sensor zu erfassenden Parameter.

Mindestens auf der dem Sensor abgewandten Seite weist der Messbereich des erfindungsgemäßen Sensorsystems eine erodierbare Schutzbeschichtung auf. Die erodierbare Schutzbeschichtung kann zwei oder mehr erodierbare Schichten aufweisen oder daraus bestehen, wobei die einzelnen Schichten der Schutzbeschichtung gleichartig oder unterschiedlich sein können. Die erodierbare Schutzbeschichtung ist dabei durchlässig für die vom Sensor zu erfassenden Parameter. Insbesondere kann die erodierbare Schutzbeschichtung zu diesem Zweck als perforierte Oberfläche oder Membran ausgebildet sein. Bevorzugt sind die Öffnungen der Perforation derart beschaffen und angeordnet, dass die erodierbare Schutzbeschichtung durchlässig ist für die vom Sensor zu erfassenden Parameter. In einer besonderen Ausführungsform sind eine Mehrzahl oder sogar alle Öffnungen der Perforation mit einem pH-sensitiven Hydrogel gefüllt.

Die erodierbare Schutzbeschichtung kann neben dem Messbereich auch die dem Sensor abgewandte Oberfläche des Gehäuses ganz oder teilweise bedecken. In einer bevorzugten Ausführungsform ist die erodierbare Schutzbeschichtung ausschließlich auf dem Messbereich angebracht.

Unter einer erodierbaren Schutzbeschichtung wird eine Schutzbeschichtung verstanden, die im Wesentlichen unbeständig ist gegenüber den Bedingungen, des Millieus und/oder der Umgebung nach erfolgter Implantation des Sensorsystems. Unter einer Erosion wird dabei eine in einem zeitlich definierten Prozess schichtweise erfolgende Auf- bzw. Ablösung verstanden. Bevorzugt zeichnet sich die erodierbare Schutzbeschichtung dadurch aus, dass die Schutzbeschichtung zum Ende der geplanten Standzeit, des geplanten Mess- oder Erfassungszeitraums mindestens eine Schicht der Schutzbeschichtung im Wesentlichen auf- bzw. abgelöst ist. In einer anderen bevorzugten Ausführungsform ist die erodierbare Schutzbeschichtung zum Ende der geplanten Standzeit, des geplanten Mess- oder Erfassungszeitraums im Wesentlichen vollständig auf- bzw. abgelöst.

Die erodierbare Schutzbeschichtung ist vorzugsweise elektrisch erodierbar. Unter einer elektrischen Erodierbarkeit wird dabei verstanden, dass die Schutzbeschichtung nach erfolgter Implantation am Implantationsort aufgrund von elektrischen Wechselwirkungen abgetragen wird. Dies ist insbesondere dann der Fall, wenn das erfindungsgemäße Sensorsystem derart ausgebildet ist, dass die erodierbare Schutzbeschichtung als Opferanode fungiert.

Bevorzugt umfasst oder besteht die erodierbare Schutzbeschichtung des erfindungsgemäßen Sensorsystems aus einem biokompatiblen erodierbaren Polymer, Hydrogel, Metall oder einer Mischung davon. Bevorzugte Beispiele für solche Polymere, Hydrogele und Metalle sind beispielsweise vorbehandeltes oder granuliertes oder strukturiertes Poly-L-Lactid, Parylen, MP35N, Gold, Platin/Iridium, Magnesiumlegierungen, Fe304, Hydrogel: Polymer aus Basis von Acrylamid, Methacrylamid, Dimethylaminoethylmathacrylate oder einem Derivat von Acrylamid, Methacrylamid, Dimethylaminoethylmathacrylate [3] oder poly N-Isopropylacrylamide [2] und Poly-N-isopropylacrylamide-co-allylamin und PNI-PAM mit Poly(p-dioxanon) als Hartsegment.

Besonders bevorzugt umfasst oder besteht die erodierbare Schutzbeschichtung aus einem biokorrodierbaren Werkstoff. Beispiele für einen biokorrodierbaren Werkstoff sind biokorrodierbare Polymere und/oder biokorrodierbare Metalle bzw. Legierungen wie beispielsweise Magnesiumlegierungen, Eisenlegierungen, Zinklegierungen oder biokorrodierbare Polymere wie Polyalkylenphosphat.

In einer bevorzugten Ausführungsform des erfindungsgemäßen Sensorsystems ist die erodierbare Schutzbeschichtung als Opferanode ausgebildet.

Dazu kann das Sensorsystem eine metallische Oberfläche aufweisen, die als Kathode ausgebildet ist. Die Kathode kann beispielsweise ein biokompatibles Metall oder eine biokompatible Legierung aufweisen oder daraus bestehen. Bevorzugt umfasst oder besteht die Kathode ein biokompatibles Edelmetall, Edelstahl, Titan, Gold, Platin oder eine Legierung enthaltend eines oder mehrere dieser Metalle. Die metallische Oberfläche, die als Kathode ausgebildet ist, kann als Bestandteil des Gehäuses ausgeführt sein. Insbesondere kann das Gehäuse selbst teilweise oder als ganzes als Kathode ausgeführt sein.

Dazu weist die erodierbare Schutzbeschichtung eine oder mehrere metallische Schichten auf, die als Opferanode ausgebildet sind. Um als Opferanode fungieren zu können, bestehen eine, mehrere oder alle metallischen Schichten der erodierbaren Schutzbeschichtung aus einem gegenüber der metallischen Oberfläche der Kathode unedleren Material. Bei diesem gegenüber der Kathode unedleren Material handelt es sich bevorzugt um Metalle oder Legierung. Bevorzugt handelt es sich um biokompatible Metalle oder Legierungen, besonders bevorzugt um biokompatible unedle Metalle oder Legierungen, beispielsweise um Magnesium, Eisen, Zink oder deren Legierungen.

Das erfindungsgemäße Sensorsystem kann zusätzlich eine Spannungsquelle aufweisen, die mit der metallischen Oberfläche, die als Kathode ausgebildet ist, und einer, mehreren oder allen metallischen Schichten der erodierbaren Schutzbeschichtung, die als Opferanode ausgebildet sind, elektrisch leitend verbunden ist. Bevorzugt ist die Spannungsquelle regel- und/oder steuerbar ausgestaltet, besonders bevorzugt von außen nach erfolgter Implantation regel- und/oder steuerbar. Durch Anlegen einer ausgewählten Spannung ist so die Erosionsgeschwindigkeit der erodierbaren Schutzbeschichtung gezielt beeinflussbar. Die Erosionsgeschwindigkeit der erodierbaren Schutzbeschichtung kann durch Einprägung eines kathodischen Schutzstroms verlangsamt und durch Einprägung eines anodischen Korrosionsstroms beschleunigt werden.

Figuren:
- Figur 1:: zeigt eine schematische Darstellung eines Sensorsystems gemäß dem Stand der Technik.
- Figur 2:: zeigt eine schematische Darstellung einer ersten Ausführungsform des erfindungsgemäßen Sensorsystems.
- Figur 3:: zeigt eine schematische Darstellung einer zweiten Ausführungsform des erfindungsgemäßen Sensorsystems.

Nachfolgend wird die Erfindung anhand von Ausführungsbeispielen näher erläutert.

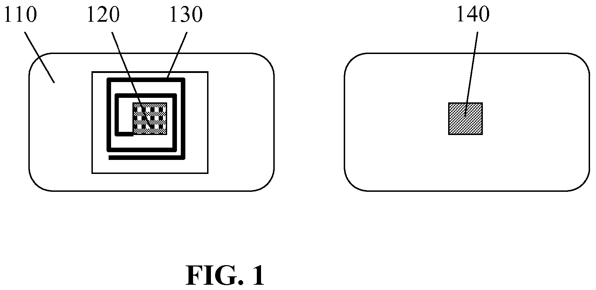

In Figur 1 ist ein Sensorsystem gemäß des Standes der Technik dargestellt, um die wesentlichen Funktionen und Elemente eines Sensorsystems zu verdeutlichen. Das implantierbare Sensorsystem besteht aus dem eigentlichen Sensor 120 und optional einer Spule 130 zur telemetrischen Sensoraktivierung und Kommunikation. Der Sensor 120 und die Spule 130 sind von einem Gehäuse 110 umschlossen, welches beispielsweise aus einem flexiblen Polymermaterial besteht. Das Gehäuse 110 weist einen Messbereich 140 auf, der in Form einer Aussparung ausgestaltet ist. Der Messbereich 140 ist durchlässig für die vom Sensor 120 zu erfassenden Parameter und dient dazu einen Kontakt zwischen dem eigentlichen Sensor 120 und den vom Sensor 120 zu erfassenden Parametern zu erlauben.

Ausgehend von der in Figur 1 dargestellten Ausführungsform gelangt man zum erfindungsgemäßen Sensorsystem dadurch, dass der Messbereich 140 mit einer erodierbaren Schutzbeschichtung versehen wird, die durchlässig ist für die vom Sensor 120 zu erfassenden Parameter.

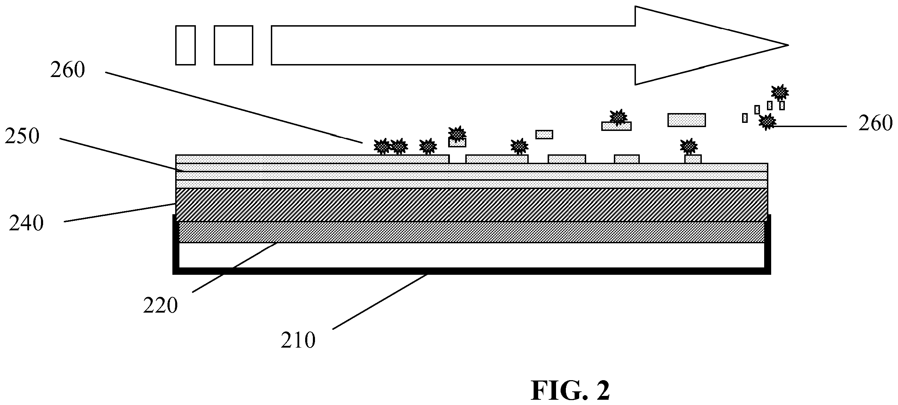

In Figur 2 ist eine erste Ausführungsform des erfindungsgemäßen Sensorsystems dargestellt. Der Sensor 220 ist im Gehäuse 210 angeordnet. Der Messbereich 240 zeichnet sich dadurch aus, dass in diesem Bereich das Gehäuse 210 eine Aussparung aufweist, die mit einer nicht-erodierbaren, für die vom Sensor 220 zu erfassenden Parameter durchlässigen Schicht bedeckt ist. Der Messbereich 240 ist auf der dem Sensor 220 abgewandten Seite mit einer erodierbaren Schutzbeschichtung 250 bedeckt, die aus mehreren erodierbaren Einzelschichten gebildet ist. Lagern sich nun beispielsweise Proteine 260 als Zeichen einer beginnenden Verkapselung auf der erodierbaren Schutzbeschichtung 250 ab, so werden diese durch die zeitabhängig fortschreitende Erosion (siehe Pfeil) der erodierbaren Schutzbeschichtung 250 abgetragen, bevor es zu einer Verkapselung des Sensorsystems kommen kann. Durch die Erosion der obersten Schicht der erodierbaren Schutzbeschichtung 250 lösen sich auch die darauf haftenden Proteine 260 ab und werden wieder freigesetzt. Dadurch wird eine Sensorbeeinträchtigung vermieden.

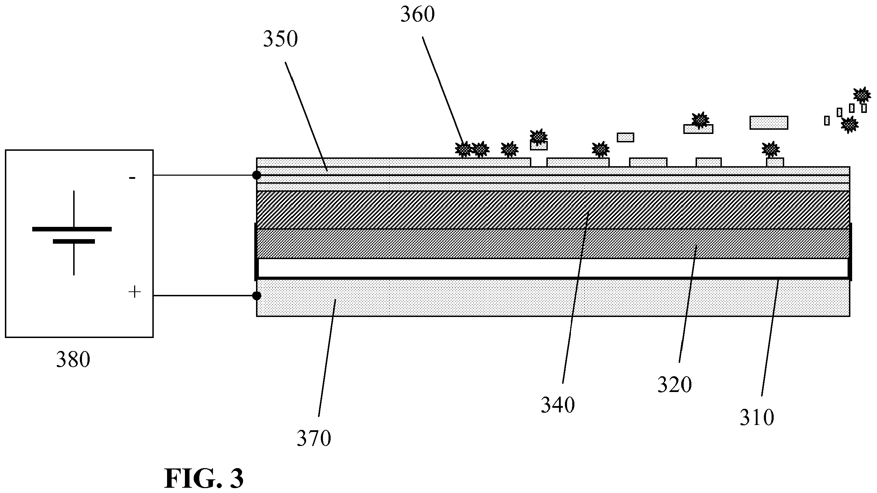

In Figur 3 ist eine zweite Ausführungsform des erfindungsgemäßen Sensorsystems dargestellt. Der Sensor 320 ist im Gehäuse 310 angeordnet. Der Messbereich 340 zeichnet sich dadurch aus, dass in diesem Bereich das Gehäuse 310 eine Aussparung aufweist, die mit einer nicht-erodierbaren, für die vom Sensor 320 zu erfassenden Parameter durchlässigen Schicht bedeckt ist. Der Messbereich 340 ist auf der dem Sensor 320 abgewandten Seite mit einer erodierbaren Schutzbeschichtung 350 bedeckt, die aus mehreren erodierbaren metallischen Einzelschichten gebildet ist. Dabei ist die erodierbare Schutzbeschichtung 350 als Opferanode ausgebildet. Dazu kann die erodierbare Schutzbeschichtung 350 aus einer oder mehreren metallischen Schichten bestehen, wobei die metallischen Schichten vorzugsweise ein biokompatibles unedles Metall oder Metalllegierung aufweisen oder daraus bestehen, z.B. eine Magnesiumlegierung. Auf dem Gehäuse 310 ist eine metallische Oberfläche 370 vorgesehen, die als Kathode ausgestaltet ist. Die Kathode enthält oder besteht vorzugsweise aus einem biokompatiblen Metall oder Metalllegierung, bevorzugt handelt es sich dabei um ein Edelmetall, besonders bevorzugt um Edelstahl, Titan, Gold oder Platin bzw. Legierungen enthalten mindestens eines dieser Metalle. Die erodierbare Schutzschicht 350 als Opferanode und die metallische Oberfläche 370 des Gehäuses 310 als Kathode stehen über eine regel- und/oder steuerbare Spannungsquelle 380 elektrisch leitend miteinander in Kontakt. Die Spannungsquelle ist in Figur 3 der Übersichtlichkeit halber außerhalb des Gehäuses 310 dargestellt. Natürlich kann die Spannungsquelle 380 auch am oder innerhalb des Gehäuses 310 angeordnet sein. Wird nun an der Spannungsquelle 380 eine ausgewählte Spannung angelegt, so kann dadurch die Erosionsgeschwindigkeit der erodierbaren Schutzbeschichtung 350 beeinflusst werden. So kann die Erosion beschleunigt, verlangsamt oder zeitweise ganz eingestellt werden. Die Regelung der Erosionsgeschwindigkeit kann beispielsweise in Abhängigkeit von einem Sensorkalibriersignals erfolgen.

Lagern sich nun beispielsweise Proteine 360 als Zeichen einer beginnenden Verkapselung auf der erodierbaren Schutzbeschichtung 350 ab, so werden diese durch regel- und/oder steuerbare Erosion der erodierbaren Schutzbeschichtung 350 mittels der Spannungsquelle 380 abgetragen, bevor es zu einer Verkapselung des Sensorsystems kommen kann. Durch die Erosion der obersten Schicht der erodierbaren Schutzbeschichtung 350 lösen sich auch die darauf haftenden Proteine 260 ab und werden wieder freigesetzt. Dadurch wird eine Sensorbeeinträchtigung regel- und/oder steuerbar vermieden.

## Patentansprüche

1. Implantierbares Sensorsystem umfassend einen Sensor (320), der in einem Gehäuse (310) angeordnet ist, wobei das Gehäuse (310) einen Messbereich (340) aufweist, der durchlässig ist für die vom Sensor (320) zu erfassenden Parameter,
wobei der Messbereich (340) mindestens auf der dem Sensor (320) abgewandten Seite eine erodierbare Schutzbeschichtung (350) aufweist, die durchlässig ist für die vom Sensor (320) zu erfassenden Parameter,
**dadurch gekennzeichnet, dass**
die erodierbare Schutzbeschichtung (350) elektrisch erodierbar und aufgrund von elektrischen Wechselwirkungen abtragbar ist, und
das Sensorsystem eine metallische Oberfläche (370) aufweist, die als Kathode ausgebildet ist, und die erodierbare Schutzbeschichtung (350) eine oder mehrere metallische Schichten aufweist oder daraus besteht, die als Opferanode ausgebildet sind, wobei die metallischen Schichten der erodierbaren Schutzbeschichtung (350) aus einem gegenüber der metallischen Oberfläche (370) der Kathode unedleren elektrisch leitenden Material bestehen.

2. Sensorsystem nach Anspruch 1, wobei der Messbereich (340) als Aussparung im Gehäuse (310) ausgebildet ist oder einen Werkstoff aufweist oder daraus besteht, der durchlässig ist für die vom Sensor (320) zu erfassenden Parameter.

3. Sensorsystem nach einem der vorhergehenden Ansprüche, wobei das Gehäuse (310) einen nicht-erodierbaren Werkstoff aufweist oder daraus besteht.

4. Sensorsystem nach einem der vorhergehenden Ansprüche, wobei die metallischen Schichten der erodierbaren Schutzbeschichtung (350) biokompatible unedle Metalle oder Legierungen aufweisen oder daraus bestehen.

5. Sensorsystem nach einem der vorhergehenden Ansprüche, wobei die metallische Oberfläche (370), die als Kathode ausgebildet ist, biokompatible Metalle oder Legierungen aufweist oder daraus besteht.

6. Sensorsystem nach einem der vorhergehenden Ansprüche, wobei die metallische Oberfläche (370), die als Kathode ausgebildet ist, Bestandteil des Gehäuses (310) ist.

7. Sensorsystem nach einem der vorhergehenden Ansprüche, wobei das Sensorsystem zusätzlich eine Spannungsquelle (380) aufweist, die mit der metallischen Oberfläche (370), die als Kathode ausgebildet ist, und einer, mehreren oder allen metallischen Schichten der erodierbaren Schutzbeschichtung (350), die als Opferanode ausgebildet sind, elektrisch leitend verbunden ist.

8. Sensorsystem nach Anspruch 7, wobei die Spannungsquelle (380) regel- und/oder steuerbar ist, so dass durch Anlegen einer ausgewählten Spannung die Erosionsgeschwindigkeit der erodierbaren Schutzbeschichtung (350) gezielt beeinflussbar ist.

## Claims

1. Implantable sensor system comprising a sensor (320) which is situated in a housing (310), the housing (310) having a measurement region which is permeable for the parameters to be detected by the sensor, wherein
the measurement region (340) has an erodible protective coating (350) which is permeable for the parameters to be detected by the sensor (320),
**characterized in that**
the erodible protective coating (350) is electrically erodible and is removable due to electrical interactions, and
the sensor system has a metallic surface (370) which is designed as a cathode, and the erodible protective coating (350) includes or is composed of one or more metallic layers which are designed as a sacrificial anode, the metallic layers of the erodible protective coating (350) being composed of an electrically conductive material which is less noble than the metallic surface of the cathode.

2. Sensor system according to claim 1, wherein the measurement region (340) is designed as a cutout in the housing (310), or contains or is composed of a material which is permeable for the parameters to be detected by the sensor (320).

3. Sensor system according to one of the preceding claims, wherein the housing (310) contains or is composed of a nonerodible material.

4. Sensor system according to one of the preceding claims, wherein the metallic layers of the erodible protective coating (350) contain or are composed of biocompatible not noble metals or alloys.

5. Sensor system according to one of the preceding claims, wherein the metallic surface (370) which is designed as a cathode contains or is composed of biocompatible metals or alloys.

6. Sensor system according to one of the preceding claims, wherein the metallic surface (370) which is designed as a cathode is a component of the housing (310).

7. Sensor system according to one of the preceding claims, wherein the sensor system also has a voltage source (380) which is connected in an electrically conductive manner to the metallic surface (370) which is designed as a cathode, and to one, multiple, or all metallic layers of the erodible protective coating (350) which are designed as a sacrificial anode.

8. Sensor system according to claim 7, wherein the voltage source (380) is regulatable and/or controllable so that the rate of erosion of the erodible protective coating (350) may be influenced in a targeted manner by applying a selected voltage.

## Revendications

1. Système de détection implantable comprenant un capteur (320), qui est disposé dans un boitier (310), le boitier (310) présentant une zone de mesure (340) qui est perméable pour les paramètres à détecter par le capteur (320),
dans lequel la zone de mesure (340) présente un revêtement de protection (350) érodable au moins sur le côté opposé au capteur (320) qui est perméable pour les paramètres à détecter par le capteur (320),
**caractérisé en ce que**
le revêtement de protection (350) érodable est érodable électriquement et peut être retiré suite à des interactions électriques, et
le système de détection présente une surface (370) métallique qui est conçue sous forme de cathode et le revêtement de protection (350) érodable présente une ou plusieurs couches métalliques ou en est constitué, qui sont conçues comme des anodes sacrificielles,
dans lequel les couches métalliques du revêtement de protection (350) érodable sont constituées d'un matériau électriquement conducteur moins noble vis-à-vis de la surface (370) métallique de la cathode.

2. Système de détection selon la revendication 1, dans lequel la zone de mesure (340) est conçue sous forme d'un évidement dans le boitier (310) ou présente une matière ou en est constitué qui est perméable pour les paramètres à détecter par le capteur (320).

3. Système de détection selon l'une des revendications précédentes, dans lequel le boitier (310) présente une matière non érodable ou en est constitué.

4. Système de détection selon l'une des revendications précédentes, dans lequel les couches métalliques du revêtement de protection (350) érodable présentent des métaux ou des alliages non nobles biocompatibles ou en sont constituées.

5. Système de détection selon l'une des revendications précédentes, dans lequel la surface (370) métallique qui est conçue sous forme de cathode, présente des métaux ou des alliages biocompatibles ou en est constituée.

6. Système de détection selon l'une des revendications précédentes, dans lequel la surface (370) métallique qui est conçue sous forme de cathode est un composant du boitier (310).

7. Système de détection selon l'une des revendications précédentes, le système de détection présentant en outre une source de tension (380) qui est reliée de manière conductrice électriquement avec la surface (370) métallique qui est conçue sous forme de cathode et une, plusieurs ou toutes les couches métalliques du revêtement de protection (350) érodable, qui sont conçues comme des anodes sacrificielles, sont reliées de manière conductrice électriquement.

8. Système de détection selon la revendication 7, dans lequel la source de tension (380) peut être régulée et/ou commandée, de sorte que par l'application d'une tension choisie, la vitesse d'érosion du revêtement de protection (350) érodable puisse être influencée de manière ciblée.
